(19) Europäisches Patentamt
European Patent Office
Office européen des brevets

(11) EP 2 314 277 B1

(12) # EUROPEAN PATENT SPECIFICATION

(45) Date of publication and mention of the grant of the patent:
**19.04.2017 Bulletin 2017/16**

(21) Application number: **09802672.7**

(22) Date of filing: **24.07.2009**

(51) Int Cl.:
***A61K 8/60*** (2006.01) ***A61Q 17/04*** (2006.01)
***A61Q 19/00*** (2006.01) ***A61Q 19/08*** (2006.01)

(86) International application number:
**PCT/JP2009/003489**

(87) International publication number:
**WO 2010/013423 (04.02.2010 Gazette 2010/05)**

(54) **EXTERNAL PREPARATION FOR SKIN, AND WRINKLE-REPAIRING AGENT**

EXTERNE ZUBEREITUNG FÜR DIE HAUT UND FALTENKORREKTURMITTEL

PRÉPARATION EXTERNE POUR LA PEAU, ET AGENT DE RÉPARATION DES RIDES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **28.07.2008 JP 2008194068**

(43) Date of publication of application:
**27.04.2011 Bulletin 2011/17**

(73) Proprietor: **Kao Corporation**
**Chuo-Ku**
**Tokyo 103-8210 (JP)**

(72) Inventors:
- **MIURA, Kyoko**
  **Odawara-shi**
  **Kanagawa 250-0002 (JP)**
- **HARATAKE, Akinori**
  **Odawara-shi**
  **Kanagawa 250-0002 (JP)**

(74) Representative: **Hoffmann Eitle**
**Patent- und Rechtsanwälte PartmbB**
**Arabellastraße 30**
**81925 München (DE)**

(56) References cited:
**WO-A1-01/37808**
**DE-A1- 19 911 053**
**JP-A- 4 273 818**
**JP-A- 6 219 947**
**JP-A- 63 218 630**
**JP-A- S63 218 630**
**US-A1- 2006 275 229**

- **OGISO, T ET AL.: "Mechanism of the enhancement effect of n-octyl-beta-D-thioglycoside on the transdermal penetration of...", JOURNAL OF PHARMACEUTICAL SCIENCES, vol. 83, no. 12, 1 December 1994 (1994-12-01), pages 1676-1681, XP55085720,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 314 277 B1

**Description**

Field of the Invention

The present invention relates to an external preparation for skin and a wrinkle-reducing agent.

Background of the Invention

Organs of all living organisms including humans grow from birth, then gradually lose their functions with age, and become dysfunctional at some time. When the proportion of the organs which have become dysfunctional exceeds a certain extent, the living organisms die. The process in which the functions become gradually lost is called aging. Skin is directly affected by surroundings and has important functions of maintaining the internal condition of a living body. It is therefore rare that the skin becomes completely dysfunctional. However, the skin is an organ on which aging signs such as wrinkles, liver spots, dullness, and sags are liable to appear, and those signs are particularly remarkable in a site that is exposed to sun light.

The progression of skin aging weakens an ability to defend against irritation such as oxidation stress and causes the disturbance of the skin internal condition to further progress aging. In general, histological changes in the skin with age differ in an exposed site and an unexposed site from each other and are classified into photoaging and physiological aging (Non Patent Document 1). In particular, the exposed site is always exposed to strong oxidation stress such as ultraviolet radiation and hence the progression of aging is remarkable. Photoaged skin leads to cosmetically undesirable conditions such as thickened epidermis and deep and large wrinkles. On the other hand, in physiological aging, it has been pointed out that so-called fine wrinkles appear and the moist condition of the horny layer has a high correlation with the development of such fine wrinkles (Non Patent Document 1), and that the horny layer becomes thick whereas the epidermis becomes thin (Non Patent Documents 1 and 2).

It is considered that epidermal thickening and wrinkles caused by photoaging cannot be sufficiently prevented and reduced by merely protecting skin from drying using a moisture-retaining agent or the like. In the United States, retinoic acid, which serves as a substance having an effect of reducing wrinkles resulting from the progression of photoaging, has been used as a prescription drug. However, retinoic acid has not been approved in Japan yet because of its strong adverse effects and safety concerns (Non Patent Document 3). Thus, there is a demand for a wrinkle-reducing substance which is high in safety and has a sufficient effect.

Meanwhile, it is known that an alkyl thioglycoside may be applied as a micelle surfactant into, for example, oral pharmaceuticals such as an oral agent for the treatment of diabetes (Patent Document 1) and may be employed as a membrane protein solubilizer (Patent Documents 2 and 3), and n-octyl-β-D-thio-glucopyranoside may be employed as a transdermal absorption promoter of a drug (Patent Document 4). However, no studies have been made on actions in the case of the application to skin, in particular, actions on wrinkles.

Prior art Document

Patent Document

[Patent Document 1] JP-A-2002-69000
[Patent Document 2] JP-A-61-7288
[Patent Document 3] JP-A-05-32689
[Patent Document 4] JP-A-63-218630

Non Patent Document

[Non Patent Document 1] Kiichiro Danno, "Photoaging and wrinkles", "Fragrance Journal", Fragrance Journal Ltd., issued on April 15, 1998, 26(4), pp. 11-17
[Non Patent Document 2] Setsuko Jitsukawa, Kazushige Hara, "A new xylose-derived active cosmetic ingredient for anti-aging - Toward sustainable development", "Fragrance Journal", Fragrance Journal Ltd., issued on October 15, 2006, 34(10), pp. 35-39
[Non Patent Document 3] Yoshio Hamada, Gen Fukuse, "Retinoids as anti-wrinkle treatment", "Fragrance Journal", Fragrance Journal Ltd., issued on April 15, 1998, 26(4), pp. 75-77

Ogiso T. et al., J. Pharm. Sci. 83(12), 1676 (1994), examine the enhancement effect of n-octyl-beta-D-thioglycoside on the transdermal penetration of active ingredients.

WO 01/37808 A1 relates to a pharmaceutical composition comprising a solid carrier, which in turn includes a substrate and an encapsulation coat containing an active ingredient and a hydrophilic surfactant. Alkyl thioglycosides are mentioned as an example for the hydrophilic surfactant.

DE 199 11 053 A1 describes cosmetic and/or pharmaceutic compositions comprising water-soluble beta-1,3-glucanes and nucleic acids, which are said to be useful for preventing wrinkle formation of the skin due to excessive sunlight.

US 2006/275229 A1 concerns a skin care active complex comprising a vitamin A derivative, a hydroxamate derivative, an anti-inflammatory compound and optionally vitamin K.

## Summary of the Invention

### Problems to be solved by the Invention

The present invention relates to an external preparation for skin and a wrinkle-reducing agent exhibiting an excellent effect of reducing wrinkles that are remarkably evident particularly in an exposed site along with aging and exhibiting an excellent effect of keeping cosmetically healthy skin.

### Means for solving problems

In view of the above-mentioned circumstances, the inventors of the present invention have made extensive studies. As a result, the inventors have confirmed that an alkyl thioglycoside, when being externally applied to skin, exhibits effects of reducing skin wrinkles, in particular, wrinkles that remarkably appear in an exposed site and keeping cosmetically healthy skin, and has excellent safety. Thus, the present invention has been completed.

In other words, the present invention provides an external preparation for skin, including an alkyl thioglycoside represented by the general formula (1) : G-$SR_1$ (1) (in the formula: G represents a sugar selected from a monosaccharide or a disaccharide, $R_1$ represents an alkyl group having 10 to 18 carbon atoms, and G and an $SR_1$ group are bonded to each other via a β-glycosidic bond.

The present invention also concerns the use of an agent for reducing wrinkles due to photoaging, the agent including an alkyl thioglycoside represented by the general formula (2) : G-$SR_2$ (2) (in the formula, G represents a sugar selected from a monosaccharide or a disaccharide, $R_2$ represents an alkyl group having 8 to 18 carbon atoms; and G and an $SR_2$ group are bonded to each other via a β-glycosidic bond).

The present invention also provides a method of reducing wrinkles due to photoaging of skin, including applying to the skin an alkyl thioglycoside represented by the general formula (2) : G-$SR_2$ (2) (in the formula, G represents a sugar selected from a monosaccharide or a disaccharide, $R_2$ represents an alkyl group having 8 to 18 carbon atoms; and G and an $SR_2$ group are bonded to each other via a β-glycosidic bond).

### Effects of the Invention

The present invention can provide an external preparation for skin and a wrinkle-reducing agent exhibiting excellent effects of reducing wrinkles markedly formed in an exposed site along with aging and keeping the skin dermatologically and cosmetically healthy.

The invention is defined by the claims.

### Brief Description of Drawing

[FIG. 1] FIG. 1 is a graph illustrating a rate of decrease in water content (average value for n=3) of lauryl thiogalactoside solution.

### Detailed Description of the Invention

An alkyl thioglycoside to be used in the present invention is represented by the general formula (1) or (2). A sugar residue represented by G is a monosaccharide or a disaccharide, preferably, for example, glucose, galactose, lactose, or maltose, more preferably glucose or galactose.

Further, an alkyl chain having 10 to 18 carbon atoms, preferably 10 to 12 carbon atoms may be used as an alkyl chain represented by $R_1$ serving as an aglycone moiety. Moreover, an alkyl chain having 8 to 18 carbon atoms, preferably 8 to 12 carbon atoms may be used as an alkyl chain represented by $R_2$. Specific examples of the alkyl chain

include octyl (C8), ethylhexyl (C8), decyl (C10), lauryl (C12), myristyl (C14), palmityl (C16), stearyl (C18), isostearyl (C18), and oleyl (C18). The above-mentioned range is preferred because an external preparation for skin, to which the alkyl chain is applied, is excellent in terms of solubility and the like, and is also excellent in terms of ease of synthesis and physical properties.

In addition, any of linear and branched and saturated and unsaturated alkyl chains may be used as the alkyl chain. Of those, a saturated and linear chain is preferred.

More preferred examples of the alkyl thioglycoside include a $C_8$ to $C_{12}$ alkyl thioglucoside and a $C_8$ to $C_{12}$ alkyl thiogalactoside. To be specific, octyl thioglucoside, decyl thioglucoside, lauryl thioglucoside, octyl thiogalactoside, decyl thiogalactoside, and lauryl thiogalactoside are more preferred.

In addition, lauryl thioglucoside represented by the following formula (3), lauryl thiogalactoside represented by the formula (4), and octyl thioglucoside represented by the formula (5) are even more preferred.

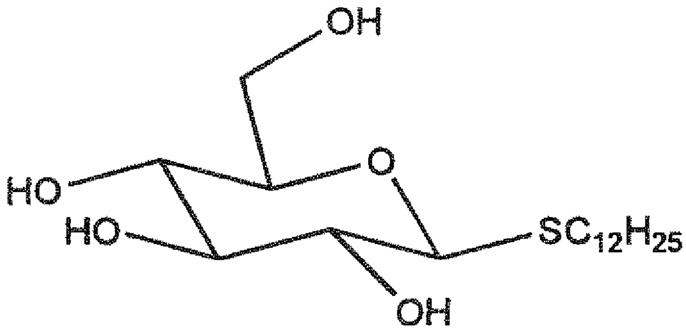
(3)

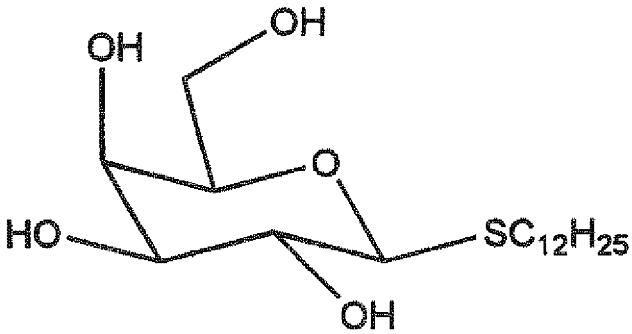
(4)

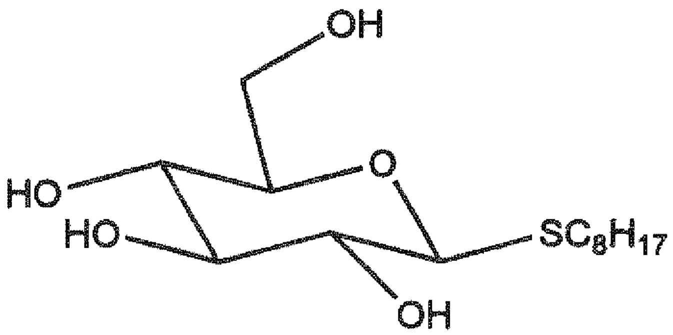
(5)

When the alkyl thioglycoside represented by the above-mentioned general formula (1) or (2) is used as a wrinkle-reducing agent for reducing wrinkles due to photoaging, the alkyl group represented by $R_2$ in the general formula (2) has 8 to 18 carbon atoms. On the other hand, when the alkyl thioglycoside is used as an external preparation for skin, the alkyl group represented by $R_1$ in the general formula (1) has 10 to 18 carbon atoms.

A synthesis method for the alkyl thioglycoside represented by the general formula (1) or (2) is, for example, a method described below.

For example, in a synthesis method for the above-mentioned structural formula (4), the alkyl thioglycoside may be produced by allowing commercially available 1,2,3,4, 6-penta-O-acetyl-D-galactopyranose as a starting material to react with 1-dodecanethiol and boron trifluoride etherate ($BF_3 \cdot OEt_2$) in a chloroform solvent and then subj ecting the resultant to a deacetylation treatment.

In the above-mentioned synthesis method, a variety of alkyl thioglycosides represented by the general formula (1) or (2) may be obtained by using, for example, 1,2,3,4,6-penta-O-acetyl-D-glucopyranose in place of 1,2,3,4,6-penta-O-acetyl-D-galactopyranose, and using, for example, commercially available 1-octanethiol, 1-nonanethiol, 1-decanethiol, 1-undecanethiol, 1-tetradecanethiol, 1-pentadecanethiol, 1-hexadecanethiol, or 1-octadecanethiol in place of 1-dodecanethiol.

Further, a commercially available alkyl thioglycoside may be purchased and used. For example, decyl 1-thio-β-D-glucopyranoside and octyl 1-thio-β-D-glucopyranoside (manufactured by SIGMA-ALDRICH) may be used.

When the alkyl thioglycoside represented by the general formula (1) or (2) is applied to skin as described in test examples and examples described later, the alkyl thioglycoside has actions of suppressing the progression of skin wrinkles, in particular, wrinkles due to photoaging, and reducing wrinkle symptoms. Accordingly, the application of a composition containing the alkyl thioglycoside to the skin can reduce wrinkles. Further, the composition containing the alkyl thioglycoside is useful as an external preparation for skin for reducing wrinkles.

The content of the alkyl thioglycoside (1) or (2) in the external preparation for skin or the wrinkle-reducing agent of the present invention is preferably 0.001 to 10 mass% (hereinafter, simply referred to as %), more preferably 0.01 to 5% on the basis of the total amount of the external preparation for skin or the wrinkle-reducing agent. A content within the above-mentioned range provides a sufficient wrinkle-reducing effect.

The form of the external preparation for skin or the wrinkle-reducing agent of the present invention may be any form as long as the form is applicable to skin. Examples of the form include solutions, milky lotions, creams, lotions, gels, packs, and bath agents.

It should be noted that the external preparation for skin and the wrinkle-reducing agent of the present invention may appropriately contain, in addition to the above-mentioned components, for example, dyes, perfumes, preservatives, surfactants, pigments, anti-oxidants, skin-whitening agents, moisture-retaining agents, ultraviolet absorbing agents, ultraviolet scattering agents, oily components, thickeners, alcohols, organic acids, pH adjustors, or water in such a range that the object of the present invention is achieved.

## Examples

Hereinafter, the present invention is described in detail based on production examples, examples, and comparative examples.

### Production Example 1 [Method of producing lauryl thioglucoside represented by formula (3)]

To a solution obtained by dissolving 1,2,3,4,6-penta-O-acetyl-D-glucopyranose (1 g) in chloroform (10 mL) were added 1-dodecanethiol (0.57 g) and $BF_3 \cdot OEt_2$ (1.6 mL), and the mixture was stirred. After the completion of the reaction, the reaction solution was washed with a sodium hydrogen carbonate aqueous solution, and then, a chloroform layer was dried over anhydrous magnesium sulfate. Subsequently, a solvent was distilled off under reduced pressure. The resultant residue was subjected to a deacetylation reaction using sodium methylate in a methanol solvent toaffordlaurylthioglucoside (0.68g, totalyield: 72%) ofinterest.

### Production Example 2 [Method of producing lauryl thiogalactoside represented by formula (4)]

To a solution obtained by dissolving 1,2,3,4,6-penta-O-acetyl-β-D-galactopyranose (1 g) in chloroform (10 mL) were added 1-dodecanethiol (0.57 g) and $BF_3 \cdot OEt_2$ (1.5 mL), and the mixture was stirred. After the completion of the reaction, the reaction solution was washed with a sodium hydrogen carbonate aqueous solution, and then, a chloroform layer was dried over anhydrous magnesium sulfate. Subsequently, a solvent was distilled off under reduced pressure. The resultant residue was subjected to a deacetylation reaction using sodium methylate in a methanol solvent to afford lauryl thiogalactoside (0.56 g, total yield: 60%) of interest.

### Moisture retention test example

In accordance with the following test method, the rate of decrease in water content of lauryl thiogalactoside (compound of Production Example 2) solution was calculated to evaluate moisture-retaining property.

(1) Test methods

0.5 g of a sample prepared by adjusting a concentration of lauryl thiogalactoside to 10 mass% with ion exchanged water was weighed in a container made of a resin to measure an initial weight (W0).

Next, the sample was left to stand still in a silica gel desiccator (RT; 25°C, RH; 20%) to measure a time-dependent weight (Wt) of the sample.

After the completion of the test, the sample was dried in a dryer (50°C) for 2 hours and then left to stand still in a silica gel desiccator for 2 hours to measure a dry weight (Ws).

The same operation was conducted for a control sample prepared using water alone, and the rate of decrease in water content was calculated with the following equation. FIG. 1 shows the results.

(2) Calculation equation

$$\cdot \text{Rate of decrease in water content (\%)} = Ht/H0 \times 100$$

(Ht=Wt-Ws, H0=W0-Ws)

FIG. 1 revealed that lauryl thiogalactoside did not suppress water volatilization in a dry state and exhibited an insufficient moisture-retaining action.

Wrinkle reduction test example

A wrinkle-reducing effect in the case of applying a sample containing a base alone or lauryl thiogalactoside (compound of Production Example 2) to photoaged skin was examined by the following test methods.

(1) Experimental animals

Ten hairless mice, which were 10 weeks old at the start of the test, were used for each group.

(2) Measurement of wrinkle-reducing effect

(2)-1. Photoaging conditions and measurement methods

Photoaging was induced by irradiation with UVA and UVB once a day, five times aweek for 8 weeks. An irradiation dose was increased every week from 20 $J/cm^2$ to 25 $J/cm^2$ and to 30 $J/cm^2$ for UVA, from 20 $mJ/cm^2$ to 30 $mJ/cm^2$ and to 40 $mJ/cm^2$ for UVB, and the maximum dose was irradiated in Week 3 or later.

A wrinkle-reducing effect was evaluated by a wrinkle score and an epidermal thickness. The wrinkle score was determined in accordance with the method of Bissett et al. (Photochem. Photobiol. 46: 367-378, 1987). In other words, the size and depth of wrinkles were comprehensively evaluated with naked eyes and scored with the following four grades : 3, "large and deep wrinkles can be confirmed" ; 2, "wrinkles can be confirmed"; 1, "no wrinkles can be confirmed"; and 0, "normal skin texture can be observe". The measurement of the epidermal thickness was performed by collecting the whole layer skin, and preparing a skin section in accordance with a conventional method, then subjecting the skin section to hematoxylin-eosin staining, and measuring the epidermal thickness with image analysis software (Microanalyzer manufactured by Nihon Poladigital, K.K.).

(2)-2. Samples and experimental methods

A sample containing lauryl thiogalactoside in an amount of 1% in a 50 vol% ethanol aqueous solution (base) was prepared (Example 1). Further, a sample containing a base alone was prepared as Comparative Example 1. First, 0.1 mL of each of those samples was applied onto the dorsal skin (about 2.5 cm in diameter) of hairless mice with a frequency of once a day, five times a week, from Week 5 after the start of UV irradiation to Week 4 after the completion of the irradiation. Subsequently, after the completion of the application, the wrinkle score was determined. The mice had been sacrificed, and the skin was then collected. Both of the wrinkle score and the epidermal thickness were compared to those of a base-applied group as a control. Tables 1 and 2 show the results.

[Table 1]

(Wrinkle score evaluation results)

| Group | | Wrinkle score |
|---|---|---|
| Example 1 | Lauryl thiogalactoside-containing sample-applied group | 2.4±0.2** |

(continued)

(Wrinkle score evaluation results)

| Group | | Wrinkle score |
|---|---|---|
| | | (p=0.0071) |
| Comparative Example 1 | Base sample-applied group | 2.8±0.1 |

(The values are each calculated as an average value ± a standard error, and the statistically significant difference was verified by a Dunnett's multiple comparison test.)

Table 1 revealed that Example 1 showed a significantly low wrinkle score value as compared to Comparative Example 1, and lauryl thiogalactoside was thus effective for wrinkles induced by photoaging.

[Table 2]

(Epidermal thickness measurement results)

| Group | | Epidermal thickness (μm) |
|---|---|---|
| Example 1 | Lauryl thiogalactoside-containing sample-applied group | |
| | | 25.94±1.61** |
| | | (p=0.0062) |
| Comparative Example 1 | Base sample-applied group | 35.38±2.77 |

(The values were each calculated as an average value ± a standard error, and the statistically significant difference was verified by a Dunnett's multiple comparison test.)

Table 2 demonstrated that the wrinkle-reducing agent-applied group of Example 1 showed a significantly thin epidermal thickness as compared to that of the applied group of Comparative Example 1, and lauryl thiogalactoside had an effect of alleviating the epidermal thickening owing to photoaging. It should be noted that, when retinoic acid is applied in this test system, retinoic acid is effective for the wrinkle score but has a function of enhancing the thickening with regard to the epidermal thickness. This action has been one factor of safety concerns. In contrast, lauryl thiogalactoside does not have any such action and also does not have any problem in an ordinary safety test.

The test results reveal that the wrinkle-reducing agent containing lauryl thiogalactoside of Example 1 clearly has an effect of reducing wrinkles due to photoaging as compared to that of Comparative Example 1 and the effect is not based on the moisture-retaining action of lauryl thiogalactoside.

Example 2

In this example and comparative example, skin creams having the following compositions were prepared in accordance with a preparation method described below. The skin creams were used as samples and evaluated for their wrinkle-reducing effects in accordance with the following procedure.

To five healthy volunteers (females, 43 to 56 years old) who, in questionnaires before the test, mentioned wrinkles at the outer corners of the eyes as a skin problem, the skin cream of Example 2 or Comparative Example 2 was applied. Then, a questionnaire survey on the condition of the skin (wrinkles) at the outer corners of the eyes was carried out in accordance with the following method. Each sample was applied onto the wrinkle portion of any one of the right or left outer corners of the eyes (about 4 $cm^2$, 2×2 cm around the outer corner of the eye for each sample) in an unit dose of about 0.2 mL twice a day, after face washing in the morning and after bathing in the evening for two consecutive months (60 days) . Next, the volunteers answered questionnaires on the conditions of the skin (wrinkles) at the right and left outer corners of the eyes after the completion of the final application.

[Table 3]

Composition of skin cream

| Raw material component | Blending amount (%) |
|---|---|
| Component A | |
| Beeswax | 2.0 |
| Stearic acid | 5.0 |
| Stearyl alcohol | 5.0 |
| Hydrogenated lanolin | 2.0 |

(continued)

Composition of skin cream

| Raw material component | Blending amount (%) |
|---|---|
| Squalene | 20.0 |
| Sorbitan monostearate | 3.0 |
| Polyoxyethylene (20) sorbitan monostearate | 3.0 |
| Propylene glycol | 5.0 |
| Component B | |
| Methylparaben | 0.2 |
| Purified water | Balance |
| Component C | |
| Lauryl thioglucoside (compound of Production Example 1) | 1.0 (Example 2) or 0 (Comparative Example 2) |
| Total | 100 |

Preparation method

Each skin cream was prepared by adding lauryl thioglucoside as the component C to the component B, dissolving each of the components A and B by heating to 80°C, and then cooling the resultant to 30°C while mixing and stirring.

Based on the questionnaire results, in each of items concerning the conditions of skin (wrinkles), the number of persons who answered that the skin cream of Example 2 was more effective than that of Comparative Example 2 are shown below.

[Table 4]

| Item Number of persons (persons) | |
|---|---|
| Wrinkles became less noticeable | 5 |
| Skin became soft | 3 |
| Skin became elastic | 4 |
| Skin became shiny | 4 |
| Skin become bright | 4 |

The test results reveal that the skin cream of Example 2 clearly reduced wrinkles and further improved suppleness and elasticity of skin, which were deteriorated by photoaging, as compared to that of Comparative Example 2. Further, no skin abnormality such as irritation or itching due to the skin cream of the present invention was observed.

Example 3

A skin lotion having the following composition was prepared in accordance with a conventional method. The skin lotion was used for 1 month by 20 healthy volunteers (female, 43 to 56 years old) who, in questionnaires before the test, mentioned wrinkles at the outer corners of the eyes as a skin problem, to perform a questionnaire survey.

[Table 5]

Composition of skin lotion

| Raw material component | Blending amount (%) |
|---|---|
| Ethanol | 8.0 |
| POE (60) hydrogenated castor oil | 0.3 |
| Polyoxyethylene (20) sorbitan monolaurate | 0.1 |
| Glycerin | 1.0 |
| Polyethylene glycol 4000 | 0.1 |
| Disodium phosphate | 0.09 |

(continued)

Composition of skin lotion

| Raw material component | Blending amount (%) |
|---|---|
| Monopotassium phosphate | 0.03 |
| Disodium edetate | 0.02 |
| Methylparaben | 0.1 |
| Octyl thioglucoside (*1) | 1.0 |
| Purified water | Balance |
| Total | 100 |

*1; Octyl 1-thio-β-D-glucopyranoside manufactured by SIGMA-ALDRICH

The skin lotion of Example 3 was used by the volunteers to perform a questionnaire survey. The results are shown below. It should be noted that the results show the number of persons who answered "yes" with respect to each item, comparing the conditions before use and after use, in a questionnaire survey performed on the following items concerning the conditions of wrinkles.

[Table 6]

| Item Number of persons (number) | |
|---|---|
| Wrinkles became less noticeable | 16 |
| The size of wrinkles decreased | 15 |
| The number of wrinkles decreased | 7 |
| Wrinkles increased | 0 |

The test results reveal that almost all the persons feel that the skin lotion of Example 3 made wrinkles less noticeable as compared to the condition before use, which was based on the reduction of wrinkles due to photoaging through a decrease in the size of wrinkles rather than a decrease in the number of wrinkles. Further, no skin abnormality such as irritation or itching due to the skin lotion of the present invention was observed.

Examples 4 and 5 (milky lotion)

The milky lotion of the present invention was prepared by a conventional method in accordance with the following composition.

[Table 7]

| Raw material component | Blending amount (%) | |
|---|---|---|
| | Example 4 | Example 5 |
| Hydrogenated lecithin | 1.0 | 1.0 |
| Cholesterol | 0.5 | 0.5 |
| Squalane | 5.0 | 5.0 |
| Octyldodecyl myristate | 3.0 | 3.0 |
| Dipropylene glycol | 4.0 | 4.0 |
| 1,3-Butylene glycol | 4.0 | 4.0 |
| Glycerin | 7.0 | 7.0 |
| Diglycerin | 2.0 | 2.0 |
| Phenoxyethanol | 0.1 | 0.1 |
| Disodium edetate | 0.02 | 0.02 |
| Potassium hydroxide | q.s. | q.s. |
| Xanthan gum | 0.1 | 0.1 |
| Alkyl acrylate/methacrylate copolymer (*2) | 0.08 | 0.08 |

(continued)

| Raw material component | Blending amount (%) | |
|---|---|---|
| | Example 4 | Example 5 |
| Carboxyvinyl polymer (*3) | 0.3 | 0.3 |
| Lauryl thioglucoside | 1.0 | - |
| Stearyl thioglucoside | - | 0.5 |
| Perfume | 0.01 | 0.01 |
| Purified water | Balance | Balance |
| Total | 100 | |
| *2; PEMULEN TR-1 manufactured by Lubrizol Advanced Materials<br>*3; Synthalene L manufactured by 3V SIGMA | | |

The milky lotion showed satisfactory results in the above-mentioned test.

Examples 6 and 7 (day essence cosmetic)

The day essence cosmetic of the present invention was prepared by a conventional method in accordance with the following composition.

[Table 8]

| Raw material component | Blending amount (%) | |
|---|---|---|
| | Example 6 | Example 7 |
| Ethanol | 10.0 | 10.0 |
| Phenoxyethanol | 0.3 | 0.3 |
| Polyoxyethylene (20) sorbitan monolaurate | 0.4 | 0.4 |
| POE (60) hydrogenated castor oil | 0.8 | 0.8 |
| Methylpolysiloxane (*4) | 2.0 | 2.0 |
| Methylphenylpolysiloxane (*5) | 0.5 | 0.5 |
| Squalane | 0.5 | 0.5 |
| Disodium edetate | 0.02 | 0.02 |
| Polyethylene glycol 4000 | 6.0 | 6.0 |
| Glycerin | 10.0 | 10.0 |
| Dipropylene glycol | 4.0 | 4.0 |
| Xanthan gum | 0.04 | 0.04 |
| Carboxyvinyl polymer (*6) | 0.3 | 0.3 |
| Potassium hydroxide | q.s. | q.s. |
| Lauryl thioglucoside | 1.0 | - |
| Lauryl thiogalactoside | - | 1.0 |
| Perfume | 0.05 | 0.05 |
| Purified water | Balance | Balance |
| Total | 100 | |
| *4; Silicone KF-96A (100CS) manufactured by Shin-Etsu Chemical Co., Ltd.<br>*5; Silicone FZ-209 manufactured by Dow Corning Toray Co., Ltd.<br>*6; Carbopol 940 manufactured by Lubrizol Advanced Materials | | |

The day essence cosmetic showed satisfactory results in the above-mentioned test.

Examples 8 and 9 (sunscreen)

The sunscreen of the present invention was prepared by a conventional method in accordance with the following composition.

[Table 9]

| Raw material component | Blending amount (%) Example 8 | Example 9 |
|---|---|---|
| Ethanol | 10.0 | 10.0 |
| Octyl methoxycinnamate | 7.0 | 7.0 |
| Poly(oxyethylene/oxypropylene)me thylpolysiloxane copolymer (*7) | 2.0 | 2.0 |
| Fine titanium oxide particles | 5.0 | 5.0 |
| Zinc oxide | 5.0 | 5.0 |
| Methylcyclopolysiloxane (*8) | 10.0 | 10.0 |
| Egg yolk lecithin | 2.0 | 2.0 |
| Lauryl thioglucoside | 1.0 | - |
| Stearyl thiogalactoside | - | 0.5 |
| Perfume | 0.1 | 0.1 |
| Purified water | Balance | Balance |
| Total | 100 | |

*7; Silicone BY22-008 manufactured by Dow Corning Toray Silicone Co., Ltd.
*8; TSF405 manufactured by Momentive Performance Materials Inc.

The sunscreen showed satisfactory results in the above-mentioned test.

It should be noted that a perfume having the following perfume prescription was used as the perfume in Examples.

[Table 10]

| Perfume prescription A | | | |
|---|---|---|---|
| Component | mass% | Component | mass% |
| Terpineol | 10.00 | Vanillin | 2.00 |
| Terpinyl acetate | 2.00 | Ethyl vanillin | 0.10 |
| Cepionate | 60.00 | Muscone | 0.50 |
| Methyl dihydrojasmonate | 250.00 | Ethylene brassylate | 42.00 |
| Indole | 0.05 | 4,6,6,7,8,8-Hexameth yl-1,3,4,6,7,8-hexah ydrocyclopentabenzop yran | 60.00 |
| 2-Methyl-3-(3,4-methyl enedioxy-phenyl)-propa nal | 3.00 | Cyclopentadecanolide | 20.00 |
| Hydroxycitronellal | 20.00 | Ambrettolide | 1.00 |
| Hydroxycitronellol | 10.00 | γ-Undecalactone | 0.40 |
| p-t-Butyl-α-methylhydr ocinnamic aldehyde | 35.00 | γ-Decalactone | 0.10 |
| 4-(4-Hydroxy-4-methylpentyl)-3-cyclohexene-1-carboxyaldehyde | 75.00 | 4-(4-Hydroxyphenyl)-2-butanone | 0.50 |
| 3-Methyl-5-phenylpenta nol | 20.00 | Musk ketone | 0.10 |
| Phenylethyl alcohol | 10.00 | Skatole | 0.01 |
| α-Ionone | 10.00 | cis-Jasmone | 0.05 |
| β-Ionone | 20.00 | Phenylethyl acetate | 0.10 |
| γ-Methyl ionone | 10.00 | Civetone | 0.20 |
| Dihydro-β-ionone | 25.00 | γ-Nonalactone | 0.05 |

(continued)

| Perfume prescription A | | | |
|---|---|---|---|
| Component | mass% | Component | mass% |
| Benzyl salicylate | 150.00 | α-Santalol | 0.20 |
| cis-3-Hexenyl salicylate | 30.00 | β-Santalol | 0.20 |
| Eugenol | 0.80 | Eugenyl acetate | 0.10 |
| Cinnamic alcohol | 5.00 | α-Hexyl cinnamic aldehyde | 20.00 |
| Cinnamic aldehyde | 0.50 | α-Damascone | 0.04 |
| Guaiol acetate | 1.00 | β-Damascone | 0.02 |
| Guaiol | 0.50 | β-Damascenone | 0.01 |
| Cedrenyl acetate | 5.00 | δ-Damascone | 0.01 |
| Cedryl methyl ketone | 30.00 | Rose absolute | 0.50 |
| 6,7-Dihydro-1,1,2,3,3-pentamethyl-4(5H)-inda n | 2.00 | Rose oil | 4.50 |
| Vetiver acetate | 10.00 | Sandal wood oil | 2.00 |
| 3-Methyl-5-(2,3,3-trim ethyl-3-cyclopenten-1-yl)-pentan-2-ol | 2.00 | Labdanum absolute | 0.05 |
| 2-Ethyl-4-(2,3,3-trime thyl-3-cyclopenten-1-y 1)-2-buten-1-ol | 0.80 | Cistus absolute | 0.01 |
| Isobornyl cyclohexanol | 35.00 | Vetiver oil | 0.50 |
| Heliotropin | 10.00 | Guaiac wood oil | 0.10 |
| Coumarin | 2.00 | Total | 1000.00 |

Industrial Applicability

The composition of the present invention can be applied as the external preparation for skin and the wrinkle-reducing agent to, for example, pharmaceuticals, quasi drugs, and cosmetics, can be formed into dosage forms such as lotions, milky lotions, creams, packs, and bath agents, and is thus very useful from the viewpoint of the beauty of skin.

## Claims

**1.** An external preparation for skin, comprising an alkyl thioglycoside represented by the general formula (1):

$$G\text{-}SR_1 \qquad (1)$$

wherein G represents a sugar selected from a monosaccharide or a disaccharide, $R_1$ represents an alkyl group having 10 to 18 carbon atoms, and G and an $SR_1$ group are bonded to each other via a β-glycosidic bond.

**2.** The external preparation for skin according to claim 1, wherein the alkyl thioglycoside is a $C_{10}$ to $C_{12}$ alkyl thioglucoside or a $C_{10}$ to $C_{12}$ alkyl thiogalactoside.

**3.** The external preparation for skin according to claim 1, wherein the alkyl thioglycoside is lauryl thioglucoside or lauryl thiogalactoside.

**4.** Use of an agent for reducing wrinkles due to photoaging by external application to the skin, the agent comprising an alkyl thioglycoside represented by the general formula (2):

$$G\text{-}SR_2 \qquad (2)$$

wherein G represents a sugar selected from a monosaccharide or a disaccharide, $R_2$ represents an alkyl group having 8 to 18 carbon atoms, G and an $SR_2$ group are bonded to each other via a β-glycosidic bond.

**5.** The use according to claim 4, wherein the alkyl thioglycoside is a $C_8$ to $C_{12}$ alkyl thioglucoside or a $C_8$ to $C_{12}$ alkyl thiogalactoside.

**6.** The use according to claim 4, wherein the alkyl thioglycoside is lauryl thioglucoside, lauryl thiogalactoside, or octyl thioglucoside.

**7.** A method of reducing wrinkles due to photoaging of skin, comprising applying to the skin an alkyl thioglycoside represented by the general formula (2):

$$G\text{-}SR_2 \qquad (2)$$

wherein G represents a sugar selected from a monosaccharide or a disaccharide, $R_2$ represents an alkyl group having 8 to 18 carbon atoms, and G and an $SR_2$ group are bonded to each other via a β-glycosidic bond.

**8.** The method according to claim 7, wherein the alkyl thioglycoside is a $C_8$ to $C_{12}$ alkyl thioglucoside or a $C_8$ to $C_{12}$ alkyl thiogalactoside.

**9.** The method according to claim 7, wherein the alkyl thioglycoside is lauryl thioglucoside, lauryl thiogalactoside, or octyl thioglucoside.

**Patentansprüche**

**1.** Äußere Zubereitung für die Haut, umfassend ein Alkylthioglycosid, wiedergegeben durch die allgemeine Formel (1):

$$G\text{-}SR_1 \qquad (1)$$

worin G für einen Zucker, ausgewählt aus einem Monosaccharid oder einem Disaccharid, steht, $R_1$ für eine Alkylgruppe mit 10 bis 18 Kohlenstoffatomen steht, und G und eine $SR_1$-Gruppe über eine β-glycosidische Bindung aneinander gebunden sind.

**2.** Äußere Zubereitung für die Haut gemäß Anspruch 1, wobei das Alkylthioglycosid ein $C_{10}$- bis $C_{12}$-Alkylthioglucosid oder ein $C_{10}$- bis $C_{12}$-Alkylthiogalactosid ist.

**3.** Äußere Zubereitung für die Haut gemäß Anspruch 1, wobei das Alkylthioglycosid Laurylthioglucosid oder Laurylthiogalactosid ist.

**4.** Verwendung eines Mittels zur Verringerung von Falten durch lichtbedingte Alterung (photo aging) durch äußere Anwendung auf die Haut, wobei das Mittel ein Alkylthioglycosid, wiedergegeben durch die allgemeine Formel (2), umfasst:

$$G\text{-}SR_2 \qquad (2)$$

worin G für einen Zucker, ausgewählt aus einem Monosaccharid oder einem Disaccharid, steht, $R_2$ für eine Alkylgruppe mit 8 bis 18 Kohlenstoffatomen steht, G und eine $SR_2$-Gruppe über eine β-glycosidische Bindung aneinander gebunden sind.

**5.** Verwendung gemäß Anspruch 4, wobei das Alkylthioglycosid ein $C_8$- bis $C_{12}$-Alkylthioglucosid oder ein $C_8$- bis $C_{12}$-Alkylthiogalactosid ist.

**6.** Verwendung gemäß Anspruch 4, wobei das Alkylthioglycosid Laurylthioglucosid, Laurylthiogalactosid oder Octylthioglucosid ist.

**7.** Verfahren zur Verringerung von Falten durch lichtbedingte Alterung der Haut, umfassend das Aufbringen eines

Alkylthioglycosids, wiedergegeben durch die allgemeine Formel (2), auf die Haut:

$$G\text{-}SR_2 \qquad (2)$$

worin G für einen Zucker, ausgewählt aus einem Monosaccharid oder einem Disaccharid, steht, $R_2$ für eine Alkylgruppe mit 8 bis 18 Kohlenstoffatomen steht, und G und eine $SR_2$-Gruppe über eine β-glycosidische Bindung aneinander gebunden sind.

**8.** Verfahren gemäß Anspruch 7, bei dem das Alkylthioglycosid ein $C_8$- bis $C_{12}$-Alkylthioglucosid oder ein $C_8$- bis $C_{12}$-Alkylthiogalactosid ist.

**9.** Verfahren gemäß Anspruch 7, wobei das Alkylthioglycosid Laurylthioglucosid, Laurylthiogalactosid oder Octylthioglucosid ist.

## Revendications

**1.** Préparation externe pour la peau, comprenant un alkyl-thioglycoside représenté par la formule générale (1) :

$$G\text{-}SR_1 \qquad (1)$$

dans laquelle G représente un sucre sélectionné parmi un monosaccharide ou un disaccharide, $R_1$ représente un groupe alkyle ayant 10 à 18 atomes de carbone, et G et un groupe $SR_1$ sont liés l'un à l'autre via une liaison β-glycosidique.

**2.** Préparation externe pour la peau selon la revendication 1, dans laquelle l'alkyl-thioglycoside est un alkyl-thioglucoside en $C_{10}$ à $C_{12}$ ou un alkyl-thiogalactoside en $C_{10}$ à $C_{12}$.

**3.** Préparation externe pour la peau selon la revendication 1, dans laquelle l'alkyl-thioglycoside est un lauryl-thioglucoside ou un lauryl-thiogalactoside.

**4.** Utilisation d'un agent pour réduire des rides dues au photovieillissement par application externe à la peau, l'agent comprenant un alkyl-thioglycoside représenté par la formue générale (2) :

$$G\text{-}SR_2 \qquad (2)$$

dans laquelle G représente un sucre sélectionné parmi un monosaccharide ou un disaccharide, $R_2$ représente un groupe alkyle ayant 8 à 18 atomes de carbone, G et un groupe $SR_2$ sont liés l'un à l'autre via une liaison β-glycosidique.

**5.** Utilisation selon la revendication 4, dans laquelle l'alkyl-thioglycoside est un alkyl-thioglucoside en $C_8$ à $C_{12}$ ou un alkyl-thiogalactoside en $C_8$ à $C_{12}$.

**6.** Utilisation selon la revendication 4, dans laquelle l'alkyl-thioglycoside est un lauryl-thioglucoside, un lauryl-thiogalactoside, ou un octyl-thioglucoside.

**7.** Méthode de réduction de rides dues au photovieillissement de la peau, comprenant appliquer à la peau un alkyl-thioglycoside représenté par la formule générale (2) :

$$G\text{-}SR_2 \qquad (2)$$

dans laquelle G représente un sucre sélectionné parmi un monosaccharide ou un disaccharide, $R_2$ représente un groupe alkyle ayant 8 à 10 atomes de carbone, et G et un groupe $SR_2$ sont liés l'un à l'autre via une liaison β-glycosidique.

**8.** Méthode selon la revendication 7, dans laquelle l'alkyl-thioglycoside est un alkyl-thioglucoside en $C_8$ à $C_{12}$ ou un alkyl-thiogalactoside en $C_8$ à $C_{12}$.

**9.** Méthode selon la revendication 7, dans laquelle l'alkyl-thioglycoside est un lauryl-thioglucoside, un lauryl-thiogalactoside, ou un octyl-thioglucoside.

Fig. 1

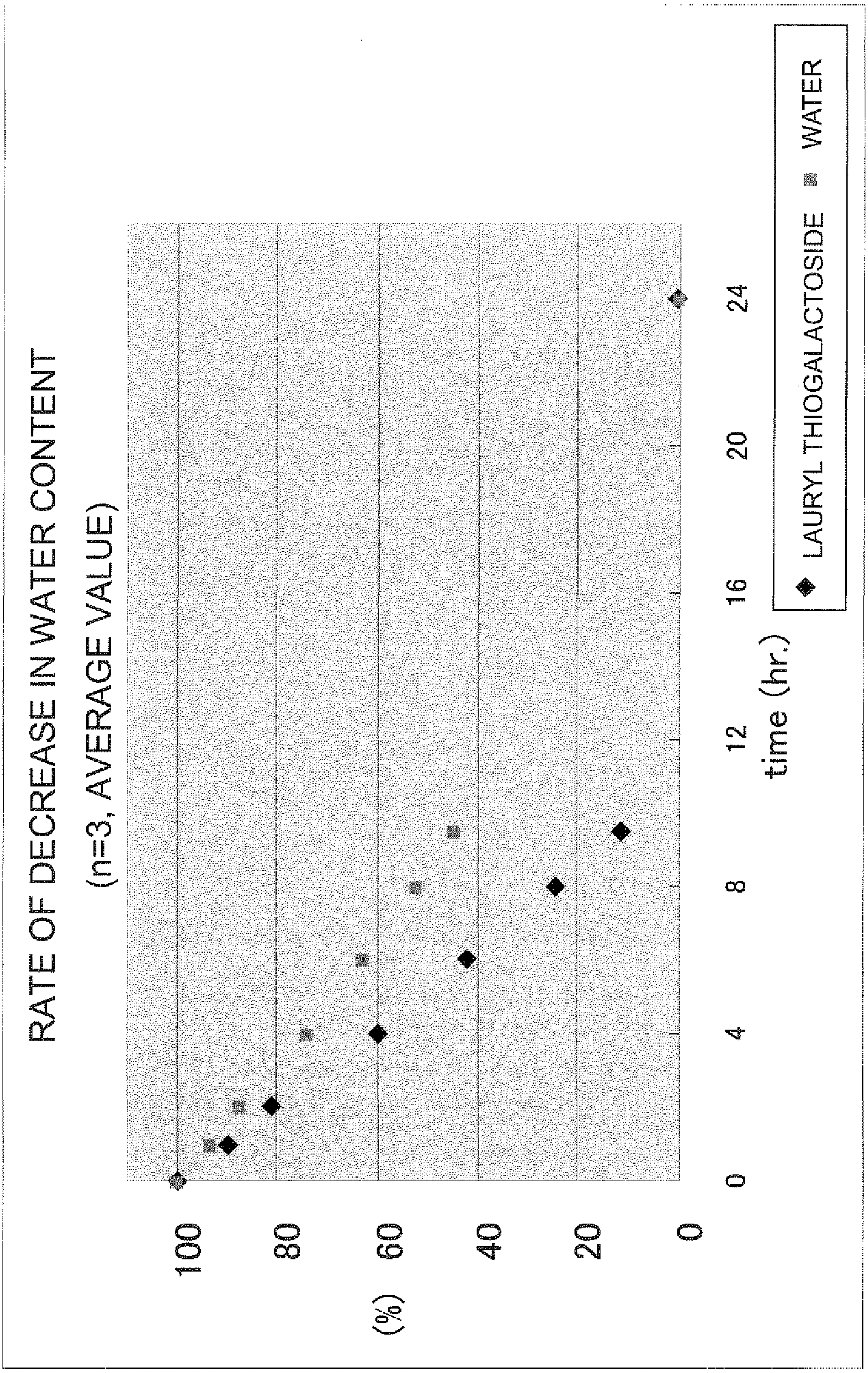
RATE OF DECREASE IN WATER CONTENT
(n=3, AVERAGE VALUE)
(%)
100
80
60
40
20
0
0
4
8
12
16
20
24
time (hr.)
LAURYL THIOGALACTOSIDE
WATER

29

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002069000 A
- JP 61007288 A
- JP 5032689 A
- JP 63218630 A
- WO 0137808 A1
- DE 19911053 A1
- US 2006275229 A1


### Non-patent literature cited in the description


- Photoaging and wrinkles. **KIICHIRO DANNO.** Fragrance Journal. Fragrance Journal Ltd, 15 April 1998, vol. 26, 11-17
- A new xylose-derived active cosmetic ingredient for anti-aging - Toward sustainable development. **SETSUKO JITSUKAWA ; KAZUSHIGE HARA.** Fragrance Journal. Fragrance Journal Ltd, 15 October 2006, vol. 34, 35-39
- Retinoids as anti-wrinkle treatment. **YOSHIO HAMADA ; GEN FUKUSE.** Fragrance Journal. Fragrance Journal Ltd, 15 April 1998, vol. 26, 75-77
- **OGISO T. et al.** *J. Pharm. Sci.,* 1994, vol. 83 (12), 1676
- **BISSETT et al.** *Photochem. Photobiol.,* 1987, vol. 46, 367-378